# EUROPEAN PATENT APPLICATION

(11) **EP 1 905 827 A1**
(43) Date of publication of application: **02.04.2008**
(21) Application number: 06019705.0
(22) Date of filing: 20.09.2006
(51) Int. Cl.: C12N 15/10

(54) **A method of detecting and/or quantifying expression of a target protein candidate in a cell, and a method of identifying a target protein of a small molecule modulator**

(71) Applicant: Institut Pasteur Korea, Seongbuk-gu Seoul 136-791 (KR)
(72) Inventor: Emans, Neil, Seoul 137-040 (KR); Nehrbass, Ulf, Yongsan Ku Seoul 140-889 (KR)
(74) Representative: Engelhard, Markus

(57) **Abstract**

The present invention relates to a method of detecting and/or quantifying expression of a target protein candidate in a cell, and to a method of identifying a target protein of a small molecule modulator.

SEQUENCE LISTING

<110>  Institut Pasteur Korea
       Emans, Neil

<120>  A method of detecting and/or quantifying expression of a target
       protein candidate in a cell, and a method of identifying a target
       protein of a small molecule modulator.

<130>  FB17777

<160>  15

<170>  PatentIn version 3.3

<210>  1
<211>  252
<212>  DNA
<213>  Drosophila melanogaster

<400>  1
agtttcaaaa tcaaaattga ggaataccaa ctagaggata aggctactta aggatcaaaa      60

aacaccaagg agacgagatt ttctaccaaa tcgagagacg aggggcaggt taatttcgtc     120

atttttggcc aagacagcaa atagaggaac agcaaagcga aaatcatttt atacctcaca     180

caacaactac acactaacta agattaggct acgcaactgt acattgtact taagtgttca     240

aagtatattt ag                                                         252

<210>  2
<211>  408
<212>  DNA
<213>  Drosophila melanogaster

<400>  2
agtttcaaaa tcaaaattga ggaataccaa ctagaggata aggctactta aggatcaaaa      60

aacaccaagg agacgagatt ttctaccaaa tcgagagacg aggggcaggt taatttcgtc     120

atttttggcc aagacagcaa atagaggaac agcaaagcga aaatcatttt atacctcaca     180

caacaactac acactaacta agattaggct acgcaactgt acattgtact taagtgttca     240

aagtatattt agtttacttt gtatataaga aaagtagcta aaagcacgcg gacagggagg     300

caggagcacc acagtcacta gccactaagc agagtcacag tcacgatcac gttcactcca     360

ggatcaggac tcggggcggg atcagcagac gctgaggaag ctgccacg                  408

<210>  3
<211>  1730
<212>  DNA
<213>  Drosophila melanogaster

<400>  3
ttcagttgtg aatgaatgga cgtgccaaat agacgtgccg ccgccgctcg attcgcactt      60

tgctttcggt tttgccgtcg tttcacgcgt ttagttccgt tcggttcatt cccagttctt     120

aaataccgga cgtaaaaata cactctaacg gtcccgcgaa gaaaaagata aagacatctc 180

gtagaaatat taaaataaat tcctaaagtc gttggtttct cgttcacttt cgctgcctgc 240

tcaggacgag ggccacacca agaggcaaga gaaacaaaaa gagggaacat aggaacagga 300

accagataat agtgacataa gcgacccttt cgcaaatatt ttggcgcaaa atgagcgggc 360

gccaagtgcc gcgtggtgga gccgcctgaa aatgacatgg aaaattcgcc gaaaatcgcg 420

cgttttggca gcatcaatcc caaagcacaa aattaatttc tatcataatt tctgggtgca 480

acacggaccc ataattgaat cgaatatagg gcttatctga tagcccggca gcaacattga 540

actttccggc tgcaaaggag acgacaccga gatcgccaat tttcgttggg ctcgttctct 600

gggctccggc gataagaaat ccatgctgat aaggacagga ggacggtctg cggcaaattg 660

aattcgattc tgacctgtat gaaagccagc ggagatacgg atacctctgg gtttatgggt 720

agaaaacgca gagcgtcgcg ccaacatcga aattatttgc gtttgcatct tctcgtcctt 780

tcgtttatcg ttctgattgc catcgtggtg gcgcggtttc tattaatttt gcttctgtat 840

cgtttgcaaa atctcaaaag attcaaaaag ttcgtcatca gcagccgcaa cacaaaaacc 900

aacgagtgta aagccgagca tacaaatatc aataaaaaca taaacattta cccaatctca 960

atctcaaaac attcgcatcg tttccacaca aatatgctta gttcgcccaa attgtgattg 1020

tatatatata tttaacggca ttaaatacaa aagattaagc cctaaattaa gtgtaaatct 1080

tacaaaacgt ctacgttttt aaacaagaaa ttgtgatatt atatattaat cgggaaattc 1140

gaagtatgag aacaaaacgg tgtatatatg taagtgggcg atgaacatca atgaatattt 1200

tagctgagca aagtacacac gaatgaatat aaatatacat gaaaatatat tttgggcacc 1260

gacttttaca ccacaattat atatcgatag aaaagacacg aaaacaatca cagaaaacta 1320

agagtttcaa aatcaaaatt gaggaatacc aactagagga taaggctact taaggatcaa 1380

aaaacaccaa ggagacgaga ttttctacca aatcgagaga cgaggggcag gttaatttcg 1440

tcatttttgg ccaagacagc aaatagagga acagcaaagc gaaaatcatt ttatacctca 1500

cacaacaact acacactaac taagattagg ctacgcaact gtacattgta cttaagtgtt 1560

caaagtatat ttagtttact ttgtatataa gaaaagtagc taaaagcacg cggacaggga 1620

ggcaggagca ccacagtcac tagccactaa gcagagtcac agtcacgatc acgttcactc 1680

caggatcagg actcggggcg ggatcagcag acgctgagga agctgccacg 1730

<210> 4
<211> 395
<212> DNA
<213> Homo sapiens

<400> 4
aattccagcg agaggcagag ggagcgagcg ggcggccggc tagggtggaa gagccgggcg 60

agcagagctg cgctgcgggc gtcctgggaa gggagatccg gagcgaatag ggggcttcgc 120

ctctggccca gccctcccgc tgatccccca gccagcggtc cgcaaccctt gccgcatcca 180

cgaaactttg cccatagcag cgggcgggca ctttgcactg gaacttacaa cacccgagca 240

aggacgcgac tctcccgacg cggggaggct attctgccca tttggggaca cttccccgcc 300

gctgccagga cccgcttctc tgaaaggctc tccttgcagc tgcttagacg ctggattttt 360

ttcgggtagt ggaaaaccag cagcctcccg cgacc 395

<210> 5
<211> 317
<212> DNA
<213> Homo sapiens

<400> 5
gtctggacgc gctgggtgga tgcggggggc tcctgggaac tgtgttggag ccgagcaagc 60

gctagccagg cgcaagcgcg cacagactgt agccatccga ggacaccccc gcccccccgg 120

cccacccgga gacacccgcg cagaatcgcc tccggatccc ctgcagtcgg cgggagtgtt 180

ggaggtcggc gccggccccc gccttccgcg ccccccacgg gaaggaagca cccccggtat 240

taaaacgaac ggggcggaaa gaagccctca gtcgccggcc gggaggcgag ccgatgccga 300

gctgctccac gtccacc 317

<210> 6
<211> 156
<212> DNA
<213> Homo sapiens

<400> 6
cgcccgtaaa atgggaacgt agaactcgtc atgacacttg tggtaaaata acattattcg 60

cttgcaaaaa acgatgtact ttgacaattt aagacacaaa aaaacacaac ctcgacaatt 120

taggagttag agaggtcgtg ttttgagatt agtgag 156

<210> 7
<211> 233
<212> DNA
<213> Homo sapiens

<400> 7
cagagatcca ggggaggcgc ctgtgaggcc cggacctgcc ccggggcgaa gggtatgtgg 60

cgagacagag ccctgcaccc ctaattcccg gtggaaaact cctgttgccg tttccctcca 120

ccggcctgga gtctcccagt cttgtcccgg cagtgccgcc ctccccacta agacctaggc 180

gcaaaggctt ggctcatggt tgacagctca gagagagaaa gatctgaggg aag 233

<210> 8
<211> 750
<212> DNA

<213>  Coxsackievirus

<400>  8
ttaaaacagc ctgtgggttg atcccaccca cagggcccat tgggcgctag cactctggta      60

tcacggtacc tttgtgcgcc tgttttatac cccctccccc aactgtaact tagaagtaac     120

acacaccgat caacagtcag cgtggcacac cagccacgtt ttgatcaagc acttctgtta     180

ccccggactg agtatcaata gactgctcac gcggttgaag gagaaagcgt tcgttatccg     240

gccaactact tcgaaaaacc tagtaacacc gtggaagttg cagagtgttt cgctcagcac     300

taccccagtg tagatcaggt cgatgagtca ccgcattccc cacgggcgac cgtggcggtg     360

gctgcgttgg cggcctgccc atggggaaac ccatgggacg ctctaataca gacatggtgc     420

gaagagtcta ttgagctagt tggtagtcct ccggcccctg aatgcggcta atcctaactg     480

cggagcacac accctcaagc cagagggcag tgtgtcgtaa cgggcaactc tgcagcggaa     540

ccgactactt tgggtgtccg tgtttcattt tattcctata ctggctgctt atggtgacaa     600

ttgagagatt gttaccatat agctattgga ttggccatcc ggtgaccaat agagctatta     660

tatatctctt tgttgggttt ataccactta gcttgaaaga ggttaaaaca ttacaattca     720

ttgttaagtt gaatacagca aaatgggagc                                      750


<210>  9
<211>  552
<212>  DNA
<213>  Encephalomyocarditis virus

<400>  9
taacgttact ggccgaagcc gcttggaata aggccggtgt gcgtttgtct atatgttatt      60

ttccaccata ttgccgtctt ttggcaatgt gagggcccgg aaacctggcc ctgtcttctt     120

gacgagcatt cctaggggtc tttcccctct cgccaaagga atgcaaggtc tgttgaatgt     180

cgtgaaggaa gcagttcctc tggaagcttc ttgaagacaa acaacgtctg tagcgaccct     240

ttgcaggcag cggaaccccc cacctggcga caggtgcctc tgcggccaaa agccacgtgt     300

ataagataca cctgcaaagg cggcacaacc ccagtgccac gttgtgagtt ggatagttgt     360

ggaaagagtc aaatggctct cctcaagcgt attcaacaag gggctgaagg atgcccagaa     420

ggtaccccat tgtatgggat ctgatctggg gcctcggtgc acatgcttta catgtgttta     480

gtcgaggtta aaaaacgtct aggccccccg aaccacgggg acgtggtttt cctttgaaaa     540

acacgatgat aa                                                         552


<210>  10
<211>  708
<212>  DNA
<213>  Cricket paralysis virus

<400>  10

tttaataagt gttgtgcaga ttaatctgca cagcactagc cacaaagttc agtggtccca 60

attatgggat ataaaactca atggtcgacg gagtgacctg gaaatgctcc ccgtgagaaa 120

ccttgtttaa acgcacttgg accttctccg gcctcagcgc caggcatagc tggatcgtca 180

atcccaagtt tggacttaga cttgctctat gttacaattg ggaggagcgt gacttaccat 240

tggtcgttga tgacgttaat tccaaacaaa aacaacaaca acaacttagt ttaaataatt 300

ccggtcttta agcatcaggg tagaccgatg aggaataaag aaccctataa gactgattcg 360

ataccaagag ctggtgtctg agggattacg actcgcgtgg tcggacagca catgttagtc 420

atgtattatg gtgtttccca acacgtatct ttgctctagt attgaacttt agtgatgagt 480

gtacttggca tgtacacctg attagttagc ccttgtggat acgccactgc cgctcaggta 540

cagtaggaca taccccaaat attgggttcc actcgtagag aagaaaatta gtggcgaaac 600

gtacggttta tgtcaaagga gaaggtgatt ttaattgcac ctgaagcaac cctaggtctt 660

ccctttgatg tgagctaata aggagtaacc gattcttaca atgtgatc 708

<210> 11
<211> 385
<212> DNA
<213> Bovine viral diarrhea virus

<400> 11
gtatacgaga actagataaa atactcgtat acatattgga caacagaaaa taactattag 60

gcctagggaa tgaatccctc tcagcgaagg ccgaaaggag gctagccatg cccttagtag 120

gactagcata atgagggggg tagcaacagt ggtgagttcg ttggatggct taagccctga 180

gtacagggta gtcgtcagtg gttcgacgcc tcggtataaa ggtctcgaga tgccacgtgg 240

acgagggcac gcccaaagca catcttaacc tgagcggggg tcgcccaggc aaaagcagat 300

cgaccaatct gttacgaata cagcctgata gggtgctgca gaggcccact gtattgctac 360

taaaaatctc tgctgtacat ggcac 385

<210> 12
<211> 373
<212> DNA
<213> Hog cholera virus

<400> 12
gtatacgagg ttagttcatt ctcgtataca cgattggaca aatcaaaatt ataatttggt 60

tcagggcctc cctccagcga cggccgaact gggctagcca tgcccatagt aggactagca 120

aaacggaggg actagccata gtggcgagct ccctgggtgg tctaagtcct gagtacagga 180

cagtcgtcag tagttcgacg tgagcagaag cccacctcga gatgctacgt ggacgagggc 240

atgccaagac acaccttaac cctagcgggg gtcgctaggg tgaaatcaca ccacgtgatg 300

ggagtacgac ctgatagggc gctgcagagg cccactatta ggctagtata aaaatctctg 360

ctgtacatgg cac 373

<210> 13
<211> 445
<212> DNA
<213> Hepatitis GB virus B

<400> 13
accacaaaca ctccagtttg ttacactccg ctaggaatgc tcctggagca ccccccctag 60

cagggcgtgg gggatttccc ctgcccgtct gcagaagggt ggagccaacc accttagtat 120

gtaggcggcg ggactcatga cgctcgcgtg atgacaagcg ccaagcttga cttggatggc 180

cctgatgggc gttcatgggt tcggtggtgg tggcgcttta ggcagcctcc acgcccacca 240

cctcccagat agagcggcgg cactgtaggg aagaccgggg accggtcact accaaggacg 300

cagacctctt tttgagtatc acgcctccgg aagtagttgg gcaagcccac ctatatgtgt 360

tgggatggtt ggggttagcc atccataccg tactgcctga tagggtcctt gcgaggggat 420

ctgggagtct cgtagaccgt agcac 445

<210> 14
<211> 585
<212> DNA
<213> Encephalomyocarditis virus

<400> 14
gcccctctcc ctcccccccc cctaacgtta ctggccgaag ccgcttggaa taaggccggt 60

gtgcgtttgt ctatatgtta ttttccacca tattgccgtc ttttggcaat gtgagggccc 120

ggaaacctgg ccctgtcttc ttgacgagca ttcctagggg tctttcccct ctcgccaaag 180

gaatgcaagg tctgttgaat gtcgtgaagg aagcagttcc tctggaagct tcttgaagac 240

aaacaacgtc tgtagcgacc ctttgcaggc agcggaaccc cccacctggc gacaggtgcc 300

tctgcggcca aaagccacgt gtataagata cacctgcaaa ggcggcacaa ccccagtgcc 360

acgttgtgag ttggatagtt gtggaaagag tcaaatggct ctcctcaagc gtattcaaca 420

aggggctgaa ggatgcccag aaggtacccc attgtatggg atctgatctg gggcctcggt 480

gcacatgctt tacatgtgtt tagtcgaggt taaaaaaacg tctaggcccc ccgaaccacg 540

gggacgtggt tttcctttga aaaacacgat gataatatgg ccaca 585

<210> 15
<211> 577
<212> DNA
<213> Encephalomyocarditis virus

<400> 15
ccccccctct ccctcccccc cccctaacgt tactggccga agccgcttgg aataaggccg 60

gtgtgcgttt gtctatatgt tattttccac catattgccg tcttttggca atgtgagggc 120

ccggaaacct ggccctgtct tcttgacgag cattcctagg ggtctttccc ctctcgccaa 180

aggaatgcaa ggtctgttga atgtcgtgaa ggaagcagtt cctctggaag cttcttgaag 240

acaaacaacg tctgtagcga ccctttgcag gcagcggaac cccccacctg gcgacaggtg 300

cctctgcggc caaaagccac gtgtataaga tacacctgca aaggcggcac aaccccagtg 360

ccacgttgtg agttggatag ttgtggaaag agtcaaatgg ctctcctcaa gcgtattcaa 420

caaggggctg aaggatgccc agaaggtacc ccattgtatg ggatctgatc tggggcctcg 480

gtgcacatgc tttacatgtg tttagtcgag gttaaaaaaa cgtctaggcc ccccgaacca 540

cggggacgtg gttttccttt gaaaaacacg atgataa 577

## Description

The present invention relates to a method of detecting and/or quantifying expression of a target protein candidate in a cell, and to a method of identifying a target protein of a small molecule modulator.

The molecular basis of many aspects of cell and protein function, in particular mammalian cell and protein function, remain elusive in many cases, and there is a need for methods that permit the exploitation of the genomic tools in the context of cell based, visual screens of protein function. This is of benefit for the identification of the protein networks operating around and within a cell biological system of interest and for the identification of the target(s) of small molecule modulators identified by chemical genetic (Eggert and Mitchison, 2006) and similar high throughput screening approaches. A current bottleneck in cell based high content screens of protein function is the identification of these targets (Peterson et al., 2006).

Moreover, in many instances, it is even difficult to reliably and quantitatively detect expression of a target protein in a cell. Some approaches in the prior art have focussed on the use of nucleic acid arrays, and the effect on the influence of external agents or pathogenic states on the expression profiles of nucleic acids within the array. This approach, in many instances, is merely qualitative at its best. Moreover, the output of such nucleic acid array experiments generates a tremendous amount of data the major part of which may be not directly amenable to further analysis. Moreover, this type of experiment only provide an insight into the expression of nucleic acids, and any inference with regard to protein expression is only indirect. Accordingly, there exists a need in the art to provide for a method of detecting and/or quantifying expression of a protein in a cell, which method is easy to perform and to analyze.

Small molecules are valuable tools to study dynamic biological processes with high temporal resolution. Small molecules may also catalyze therapeutic drug discovery. Pharmaceutically active small molecules may be discovered by screening chemical libraries for alteration of a specific protein activity in pure protein in-vitro-assays or for a desired phenotype in cell-based assays. In the past, cell-based assays have usually been established to report on effects on the activity of a single pathway or process, typically using a luminescence or fluorescence signal as readout measured in a plate reader. In cell-imaging assays, fields of cells are visualized using fluorescent tags attached to different macromolecules, and automated microscopy. Such data potentially contain a large amount of information relevant to desired as well as unexpected phenotypic changes, and the approach is sometimes called "high content screening". The potential of this method in academia and industry for the discovery and characterization of useful compounds is high, but many challenges remain, particularly at the level of target identification and data analysis.

Small molecule imaging screens typically involve a number of steps. First of all, usually cells are plated into optical-bottom-multiwell plates, treated with small molecules and incubated for an appropriate period of time. Proteins of interest are then rendered fluorescent by some combination of small molecule fluorescent probes, imunofluorescence with antibodies, and/or the expression of fluorescent protein (for example GFP)-tag proteins in cells. The latter method can be applied to both live and fixed cells. In a second step, fluorescent images are captured by automated microscopy, whereupon the images are then analyzed to provide measures of phenotypic change, identifying wells that contain desired, undesired or unexpected phenotypes. In small molecule screening methods, it is eventually necessary to identify the biochemical target causing the phenotypic change. This can be challenging, especially if phenotypic change results from perturbation of more than one macromolecule.

Consequently, there exists a need in the prior art to provide for improved methods of identifying target proteins of small molecule modulators, which methods are easy to perform and which are amenable to facile analysis. Hence it was an object of the present invention to provide for a method that permits target identification, i.e. the identification of macromolecular targets for small molecule modulators, without the need for complex biochemical assays. Moreover, it has been an object of the present invention to provide for a method which may serve as a genome wide approach for target identification which avoids labor intense biochemical methods such as those that exist in the prior art. A method is described that permits the identification of the macromolecular targets for small molecule modulators through the expression of a palette of potential target nucleic acids, preferably cDNAs, or the repression of their expression by identifying those genes that modulate the activity of the small molecule modulator. Specifically, the molecular targets of small molecules are identified through their ability to revert a small molecule phenotype or potentiate it. The method permits target identification without the need for complex biochemical assays and thus serves as a genome wide approach for target identification, in contrast to the labor intensive biochemical methods that exist in the prior art (Peterson et al 2006).

The objects of the present invention are solved by a method of detecting and/or quantifying expression of a target protein candidate in a cell comprising the steps:
- introducing a first nucleic acid encoding a marker protein into a vector,
- introducing a second nucleic acid encoding said target protein candidate the expression of which is to be detected and/or quantified, into said vector, such that said first and second nucleic acids are operably linked, such that expression of said marker protein is an indication of expression of said target protein candidate,
- introducing said vector into a cell,
- detecting and/or quantifying expression of said marker protein,
- relating said expression of said marker protein to expression of said target protein candidate, and thereby detecting and/or quantifying expression of said target protein candidate.

In one embodiment said first and second nucleic acids are operably linked within said vector by one of the following arrangements:
a) said first nucleic acid is under control of a first promoter, and said second nucleic acid is under control of a second promoter that is located separately from said first promoter, and said first and second promoters are identical in sequence or have identical activity,
b) said first nucleic acid is under control of a first promoter, and said second nucleic acid is under control of a second promoter that is located separately from said first promoter, and said first and second promoters are not identical in sequence, the activities of each of said promoters are predictable,
c) said first and said second nucleic acids are under control of a single promoter, and said first and said second nucleic acids are separated from each other by a stretch of nucleotides containing an internal ribosome entry site (IRES).

In one embodiment said marker protein is a fluorescent protein, a fragment of an antibody, an epitope, an enzyme, monomeric avidin, a peptide biotin mimic, a peptide that can be detected through direct binding, or by a peptide that can be detected through a chemical binding with or reaction with an organic molecule containing a chemical fluorophore or similar structure such that an optically detectable signal is produced.

In one embodiment said IRES is selected from IRES from viruses, IRES from cellular mRNAs, in particular IRES from picornavirus, such as polio, EMCV and FMDV, flavivirus, such as hepatitis C virus (HCV), pestivirus, such as classical swine fever virus (CSFV), retrovirus, such as murine leukaemia virus (MLV), lentivirus, such as simian immunodifficiency virus (SIV), and insect RNA virus, such as cricket paralysis virus (CRPV), and IRES from cellular mRNAs, e.g. translation initiation factors, such as eIF4G, and DAP5, transcription factors, such as c-Myc, and NF-κB-repressing factor (NRF), growth factors, such as vascular endothelial growth factor (VEGF), fibroblast growth factor 2 (FGF-2), platelet-derived growth factor B (PDGF-B), homeotic genes, such as antennapedia, survival proteins, such as X-linked inhibitor of apoptosis (XIAP), and Apaf-1, and other cellular mRNA, such as BiP.

In one embodiment
- if said first and second promoters are identical in sequence, they are selected from the group comprising CMV, EF1, SV40, human H1 and U6 promoters
- if said first and second promoters have identical activity, each of them is independently selected from the group comprising CMV, EF1, SV40, human H1 and U6 promoters
- if said first and second promoters are not identical in sequence and the activities of said promoters are predictable, each of said promoters is independently selected from the group comprising CMV, EF1, SV40, human H1 and U6 promoters
- said single promoter is selected from the group comprising CMV, EF1, SV40, human H1 and U6 promoters, and said IRES is selected from the group comprising the nucleic acids having a sequence selected from the group comprising SEQ ID NO: 1-15. In a preferred embodiment said single promoter is a CMV promoter and said IRES is from EMCV. In a particularly preferred embodiment, said promoter is the CMV immediate early (IE) promoter (pCMV_{IE})and the IRES is from EMCV; preferably said IRES from EMCV is a nucleic acid having a sequence selected from SEQ ID NO:9, 14 and 15, more preferably 14 and 15 and most preferably 14.

In one embodiment said introducing of said vector into said cell occurs by transformation, transfection, electroporation, viral transduction, transduction, ballistic delivery.

Preferably, said detecting and/or quantifying occurs by any optical detection method capable of quantitative measurement, in particular, an optical detection with a spatial resolution, microscopy, fluorescence activated cell sorting, UV-Vis spectrometry, fluorescence or phosphorescence measurements, bioluminescence measurements, wherein, more preferably, said microscopy is selected from the group comprising light microscopy, bright field microscopy, polarization microscopy, fluorescence microscopy, in particular confocal fluorescence microscopy, evanescent wave excitation microscopy, fluorescence correlation spectroscopy, fluorescence life time microscopy, fluorescence cross correlation microscopy, fluorescence recovery after photobleaching microscopy, line scanning imaging, point scanning imaging, structured illumination, deconvolution microscopy, photon counting imaging.

In one embodiment said target protein candidate and said marker protein are expressed in said cell as separate proteins.

The objects of the present invention are also solved by a method of identifying a target protein of a small molecule modulator comprising the steps:
- providing a first cell of a type that is capable of producing a signal when said first cell is exposed to a small molecule modulator, wherein said signal is a signal that can be spatially resolved and, optionally, be quantified, preferably by microscopy,
- exposing said first cell to a small molecule modulator and spatially resolving and, optionally, quantifying a first signal that is produced by said first cell as response to said small molecule modulator,
- providing a second cell of the same type as said first cell and
- performing the method according to any of claims 1-9 on said second cell,
- during performance of the method according to any of claims 1-9 on said second cell, after introducing said vector into said second cell, exposing said second cell to said small molecule modulator, and spatially resolving and, optionally, quantifying a second signal that is produced by said second cell as response to said small molecule modulator,
- comparing said first signal with said second signal, and, if there is a difference between said first signal and said second signal, attributing said difference to the expression of said target protein candidate in said second cell, thereby identifying said target protein candidate as a target protein of said small molecule modulator.

Preferably, said first signal and said second signal are optical signals that can be detected, spatially resolved, and, optionally, quantified, by microscopy, wherein, more preferably, said first signal and said second signal are fluorescence signals.

In one embodiment the expression of said marker protein produces a third signal that can be spatially resolved and distinguished from said first and second signals, wherein, preferably said third signal can be detected, spatially resolved and, optionally, quantified, by microscopy.

In a preferred embodiment said third signal is a fluorescence signal, and said first and second signals are fluorescent signals, and said third signal is spectrally distinct from said first and second signals.

In one embodiment said first signal and said second signal can only be distinguished from each other by their respective quantity.

Preferably, the method according to the present invention, for a given small molecule modulator, is performed with more than one, preferably a plurality of target protein candidates, wherein, more preferably, for a given small molecule modulator, it is performed with all possible target protein candidates of a genome of an organism.

In one embodiment the method according to the present invention is performed with more than one, preferably a plurality of small molecule modulators.

The objects of the present invention are also solved by a method of identifying a target protein of a small molecule modulator comprising the steps:
- providing a first cell of a type that is capable of producing a signal when said cell is exposed to a small molecule modulator, wherein said signal is a signal that can be spatially resolved and, optionally, be quantified, preferably by microscopy,
- exposing said first cell to a small molecule modulator and spatially resolving and, optionally, quantifying a first signal that is produced by said first cell as response to said small molecule modulator, performing this step at a number of different concentrations of said small molecule modulator to determine a first half maximum active concentration (AC₅₀) of said small molecule modulator which is the half maximum active concentration in the absence of an inhibition of expression of said target protein candidate,
- determining said first half maximum active concentration,
- providing a second cell of the same type as said first cell and
- introducing into said second cell small inhibitory RNA (siRNA) that is selected so as to inhibit expression of a target protein candidate in said second cell, preferably transfecting said second cell using small inhibitory RNA (siRNA) that is selected so as to inhibit expression of a target protein candidate in said second cell,
- exposing said second cell to said small molecule modulator and spatially resolving and, optionally, quantifying a second signal that is produced by said second cell as response to said small molecule modulator, performing this step at a number of different concentrations of said small molecule modulator to determine a second half maximum active concentration (AC₅₀) of said small molecule modulator which is the half maximum active concentration in the presence of an inhibition of expression of said target protein candidate,
- determining said second half maximum active concentration,
- comparing said first half maximum active concentration with said second half maximum active concentration, and, if there is a difference between said first half maximum active concentration and said second half maximum active concentration, attributing said difference to the inhibition of expression of said target protein candidate, thereby identifying said target protein candidate as a target protein of said small molecule modulator.

Preferably, said first and second half maximum active concentrations are half maximum inhibitory concentrations (IC₅₀), and said small molecule modulator is an inhibitor.

In another embodiment said first and second half maximum active concentrations are half maximum enhancing concentrations (EC₅₀) and said small molecule modulator is an enhancer.

It should be noted that the term "half maximum active concentration (AC₅₀)", as used herein, in some embodiments may also refer to another proportion of activity. For example, it may refer to a "90% maximum active concentration (AC₉₀)" or another percentage value that can be used as a discriminatory value, such as 60%, 70% or 80% etc. (AC₆₀, AC₇₀, AC₈₀ etc.). Again, in this case, this percentage then applies also to the terms "IC_{X}" and "EC_{X}", wherein "x" denotes the aforementioned percentage of the maximum active concentration, and wherein "IC" signifies that the small molecule modulator in this case is an inhibitor, and "EC" signifies that the small molecule modulator is an enhancer. In a preferred embodiment, the "half maximum active concentration" is a concentration with an acitivity equal to or greater than 50% of the maximum value, i.e. "AC_{≥50}".

Gene silencing is well known to someone skilled in the art and can be achieved by a plurality of methods (reviewed in Echeverri & Perrimon Nature, Reviews Genetics 2006).

Preferably, said first signal and said second signal are optical signals that can be detected, spatially resolved, and, optionally quantified, by microscopy.

More preferably, said first signal and said second signal are fluorescence signals.

The inventors have managed to devise a method that permits the identification of the macromolecular targets for small molecule modulators through the expression of a palette of potential target cDNAs, or the repression of their expression by identifying those genes that modulate the activity of the small molecule modulator. Specifically, the molecular targets of small molecules are identified through their ability to revert a small molecule phenotype or potentiate it. The method permits target identification without the need for complex biochemical assays and thus serves as a genome wide approach for target identification, in contrast to the labor intensive biochemical methods that exist in the prior art (Peterson et al 2006).

As used herein, the term "target protein candidate" is meant to refer to any protein, the expression of which one desires to detect and/or quantify. Preferably, such protein is subsequently suspected of being a target for the action of a small molecule compound. The terms "small molecule compound" and "small molecule modulator" are used synonymously and interchangeably. A "target protein candidate", as used herein, is suspected of being a target for a small molecule modulator, but such suspicion has not yet been verified. If a "target protein candidate" is identified as an actual target for a small molecule modulator, such "target protein candidate" is a "target protein" of the small molecule modulator examined.

The term "operably linked", as used herein, is meant to refer to an arrangement of two coding nucleic acids with respect to each other, such that the expression of the respective proteins coded by these nucleic acids is in a proportional relationship to each other. Hence from the detection and/or quantification of the expression of one protein encoded by one nucleic acid, one may deduce the presence and/or quantity of expression of the second protein coded by the other nucleic acid. In its simplest form, such proportional relationship may be a linear relationship, but the invention is not necessarily limited thereto.

An "internal ribosome entry site" or "IRES" as used herein, is meant to refer to a stretch of nucleotides within a coding sequence that allows for translation initiation within such coding sequence at the level of the mRNA. The protein resulting from such IRES-initiated translation is different to the protein in whose mRNA sequence it is located. Usually, in eukaryotes translation can only be initiated at the 5'-end of the mRNA molecule, since 5' cap recognition is required for the assembly of the initiation complex. It is currently believed that an IRES site mimics the 5' cap structure. Such IRES sequences were first discovered in RNA viruses, but have subsequently been identified in a number of organisms. IRES sites are located in the 5' untranslated region of RNA and allow the translation of the RNA in a cap-independent manner. A number of IRES sites have been identified, and they are characterized by their aforementioned ability to initiate translation within an mRNA in a cap-independent manner. IRES sites have been comprehensively reviewed (Martinez-Salas et al., Journal of General Virology, 2001, Vol. 82, 973-984, and Jackson, R.J., Translational Control of Gene Expression, 2000, Sonenberg et al. Eds., pp. 127-184, Cold Spring Harbor NY. Furthermore, there exists a regularly updated database for identified IRES sites under http://www.iresite.org.

Particularly preferred IRES sites that are used in the method according to the present invention are IRES from viral RNAs, such as picornavirus, flavivirus, pestivirus, retrovirus, lentivirus, and insect RNA virus, and IRES sites from cellular mRNAs, such as from translation initiation factors, transcription factors, growth factors, homeotic genes, and survival proteins. Particularly preferred IRES sites are IRES sites from picornaviruses and flaviviruses, such as EMCV and HCV. Preferred IRES sites for use in the present invention are IRES sites from Drosophila melanogaster Antennapedia, homeotic gene Antennapedia and Ultrabitorax, human c-myc Oncogene, human v-myc myelocytomatosis viral related Oncogene, human myelin transcription factor 2 (MYT2) human apoptotic protease activating factor 1 (Apaf-1), human Coxsackievirus B2 strain 20, Encephalomyocarditis virus (EMCV), Cricket paralysis virus, Bovine viral diarrhea virus 1, Hog Cholera virus (Classical swine fever virus), and Hepatitis GB virus B (GBV-B).

More specifically, an IRES site from EMCV is particularly preferred, wherein, preferably, said IRES from EMCV is a nucleic acid having a sequence selected from SE ID NO:9, 14 and 15, more preferably 14 and 15, and most preferably 14.

One idea underlying the present invention is the arrangement of an IRES-site between two nucleic acids coding for two proteins in eukaryotic mRNA molecules as bi-cistronic mRNA. In this case, such an IRES site can drive translation of the downstream protein coding region independently of the 5'-cap structure bound to the 5'-end of the mRNA molecule. In this setup, both proteins are produced in the cell, and their expression is unitarily coupled. The first protein located in the first cistron is synthesized by the cap-dependent initiation approach, while translation initiation of the second protein is directed by the IRES-site located in the intercistronic spacer region between the two protein coding regions.

Processes for introducing a nucleic acid into a cell, such as transformation, transfection, electroporation, viral transduction, transduction and/or ballistic delivery are known to someone skilled in the art and are e.g. reviewed in Sambrook and Russell, 2006.

A "small molecule modulator", as used herein, is meant to refer to a molecule which is not a protein and the molecular weight of which does not exceed 10,000. In the determination of whether a molecule is a "small molecule modulator", it may also be helpful to consider the so-called Lipinsky rules. Christopher Lipinsky searched for rules for negative drug design and formulated a rule of 5 set parameters to exclude compounds that would be unlikely to succeed due to poor absorption or permeation. Hence, according to Lipinsky's rule a molecule is not likely to be efficiently acting as a small molecule drug or modulator if it has:
1. more than 5 H-bond donors (usually the sum of NHs and OHs),
2. more than 10 H-bond acceptors (usually the sum of Ns and Os),
3. a molecular weight (MW) > 500,
4. a calculated log P >, and
5. a weak inhibition (< 100 nM) (Lipinsky et al., 1997, experimental and computational approaches to estimate solubility and permeability in drug discovery and development settings. ADV. Drug Delivery REV.23 (1-3). (P = partition coefficient of a molecule between a hydrophobic and hydrophilic environment. In departure from these Lipinsky rules, however, a "small molecule modulator", as used herein, may also have a molecular weight that is > 500 but does not exceed 10000. More specifically, a "small molecule modulator", as used herein, may however, also refer to an oligopeptide or oligonucleotide the molecular weight of which does not exceed 10,000.

A small molecule is also herein referred to as a "modulator" if such small molecule has an effect on the activity of a protein and/or cell metabolism and/or phenotype. Experimentally, small molecule modulators of proteins are often identified using high-throughput screening methods applied to chemical libraries. Such libraries may be developed in either solid or solution phase and can consist of natural compounds and their derivatives or synthetic molecules (Hall et al., 2001, J. Comb. Chem., 3:125-150). Chemical libraries are often generated using combinatorial chemistry whereby both functional groups and molecular skeletons of precursor compounds are sequentially altered (Burke et al., 2003, Science, 302:613-618; Schreiber, 2000, Science, 287:1964-1969). High-throughput screening has proven to be useful, especially in the areas of drug discovery, agrochemicals and food research.

A method frequently used in performing the method according to the present invention is microscopy. A number of types of microscopy exist and are known to someone skilled in the art. The term "microscopy" includes but is not limited to optical microscopy, confocal microscopy and automated microscopy screening methods. Particularly preferred forms of microscopy are fluorescence microscopy, in particular confocal fluorescence microscopy, light microscopy, fluorescence lifetime microscopy, fluorescence cross correlation microscopy and polarization microscopy. The limitations of microscopy are given by its resolving power which is determined by the resolution. Typically, a resolution limit is 300 nm-10 µm, preferably 300 nm-1µm, depending on the particular type of microscopy used. In this context, the term "spatially resolving a signal ", as used herein, is meant to refer to the resolution of a signal in space to a limit as low as 300 nm-10µm, preferably 300 nm-1µm, and is typically defined by the Abbé limit.
In this application, sometimes reference is made to "providing a first cell of type ..." and "providing a second cell of type ...". It will be clear to someone skilled in the art that, in each of these steps, more than one cell of a particular type may be provided, and hence, the performance of the methods according to the present invention is not restricted to the use of single cells only. In fact, many of the experiments that are performed in accordance with the method according to the present invention, will be performed on cell cultures in which there will be a plurality of cells of a particular type. Hence, "providing a first cell of type ..." and "providing a second cell of type ..." may also be substituted with "providing a first group of cells of type ..." and "providing a second group of cells of type ...".

As used herein, the term "siRNA" is meant to refer to a stretch of RNA which may be used to silence gene expression. Such siRNA may be introduced into cells in a number of ways, one of which is transfection of the cell with such siRNA. In another embodiment, however, such gene silencing may be achieved by introducing it into a cell first and allowing its expression therein so that a short hairpin RNA is actually expressed within the cell by an appropriate vector, e.g. a plasmid.

The term "half maximum active concentration" or "AC₅₀" as used herein, is meant to refer to the concentration of a small molecule modulator at which it shows its half maximum activity. For example, such small molecule modulator may be an inhibitor, in which case the activity is inhibition. Consequently, the half maximum active concentration of such small molecule inhibitor is a "half maximum inhibitory concentration" or "IC₅₀". If the small molecule modulator is an enhancer, such half maximum active concentration is a "half maximum enhancing concentration" or "EC₅₀".

It should be noted that the term "half maximum active concentration (AC₅₀)", as used herein, in some embodiments may also refer to another proportion of activity. For example, it may refer to a "90% maximum active concentration (AC₉₀)" or another percentage value that can be used as a discriminatory value, such as 60%, 70% or 80% etc. (AC₆₀, AC₇₀, AC₈₀ etc.). Again, in this case, this percentage then applies also to the terms "IC_{X}" and "EC_{X}", wherein "x" denotes the aforementioned percentage of the maximum active concentration, and wherein "IC" signifies that the small molecule modulator in this case is an inhibitor, and "EC" signifies that the small molecule modulator is an enhancer. In a preferred embodiment, the "half maximum active concentration" is a concentration with an acitivity equal to or greater than 50% of the maximum value, i.e. "AC_{≥50}".

The present inventors have found that it is possible to identify a target protein of a small molecule modulator by examining the impact of protein expression on a cell-based assay, wherein the cell-based assay is based on the exposure of cells to a small molecule modulator, as a result of which exposure a signal is produced by the cell. The outcome of such cell-based assay is measured in dependence on the extent of expression (or absence of expression) of a target protein candidate. If the expression or absence of expression of a target protein candidate has a detectable influence on the cell-based assay's results, the target protein candidate whose expression is artificially induced or increased or silenced, is a target for the small molecule modulator used in the cell-based assay.

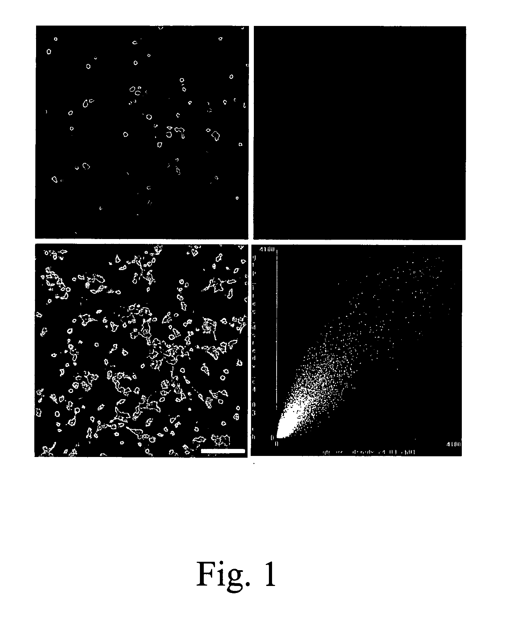

In the following, reference is made to the figures, wherein
figure 1 shows confocal laser scanning microscopy photographs of cells where green fluorescent protein and red fluorescent protein were operably linked by an IRES site, and the expression of either of them could be used as a measure of expression of the second insert in the IRES vector; panel A (top left corner) shows confocal images of GFP fluorescence in transfected cells, panel B (top right corner) confocal images of RFP fluorescence in the same cells and panel C (bottom left corner) shows a ratio image of the images in A and B representing a arbitrary pseudocolored scale for the GFP:RFP ratio. The correlation between GFP and RFP intensity per pixel is shown in panel D (bottom right corner) with GFP intensity on the X axis plotted against RFP fluorescence. The correlation between the intensities in D is 0.92 +- 0.023 (n=5) as described herein,
figure 2 shows the effect of transfection and expression of three IRES-vectors into the endothelin A-receptor-GFP cell line (GFP = green fluorescent protein); "etar" is the stable cell line transfected with an endothelin A-receptor:IRES:RFP-vector (RFP = red fluorescent protein), "etbr" is the same cell line but transfected instead with an endothelin B-receptor:IRES:RFP-vector, and "kOPr" is the same cell line but transfected with a kappa-opioid-receptor: IRES:RFP-vector (figure 2a);
figure 2B shows the same type of experiment, but this time formed with a kappa-opioid-receptor-GFP-cell line;
figure 3 shows the result of transfection of three IRES-vectors each of which had a different G-alpha-protein component in it, G-alpha s, G-alpha i and G-alpha q (Gs, Gi and Gq).
Figure 4 shows the use of the IRES approach for the identification of target proteins;
figure 4A shows the result of the effect of compound Gö6976 on the agonist induced internalization of the endothelin A-receptor; (DMSO = dimethylsulfoxide, ET-1 = endothelin-1);
figure 4B shows the results of transfection experiments of endothelin A-receptor-GFP-cells with IRES:RFP-constructs bearing either protein kinase c-alpha or protein kinase c-beta;
figure 4c shows the same results as figure 4B, but this time with the numbers of endosomes being normalized to the control at each concentration (Go = Gö 6976);
figure 5 shows the quantitation ofNF-Kappa-B nucleo-cytoplasmic transport in a high throughput immunofluorescence assay;
figure 6 shows the results of a parallel experiment, where cells were transfected with a specific siRNA or a unspecific scrambled siRNA, and leptomycin sensitive NF Kappa-B transport was measured,
figure 7 shows a schematic representation of the construct that was used in the experiments with red fluorescent protein and green fluorescent protein in example 1,

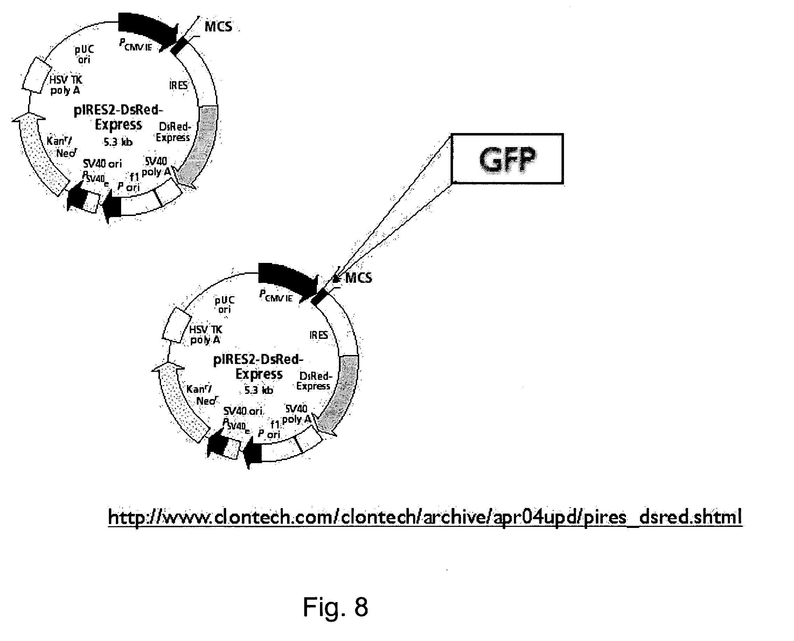

figure 8 shows an example of a commercially available vector that may be used for bicistronic expression of two protein inserts linked by an IRES site. For example the commercially available vector pIRES2-DsRed-Express-Vector commercially available from Clontech Laboratories, Inc., USA, may be used. Alternatively, the DsRed-Express-Gene i.e. the red fluorescent protein may be replaced by any other gene of interest, if upstream of the IRES a measurable/detectable protein is inserted, such as GFP or RFP. There are commercially available vectors to that extent which have multiple cloning sites upstream and downstream of the IRES site, such as the pIRES-vector of Clontech Laboratories, Inc., USA.
Figure 9 shows the effect of siRNA on the IC₅₀ of Leptomycin B on the nuclear import of NfκB when alternative SiRNAs that are mechanistically relevant are silenced under the same conditions as in figure 6.

Moreover, reference is made to the following sequences, wherein SEQ ID NO: is the IRES from NM_206445 Drosophila melanogaster Antennapedia CG 1028-RJ, transcript variantJ (Antp), mRNA. IRES name : Antp-D (1323-1574, 252bp) as referenced in Oh S. K., Scott M. P., Sarnow P. (1992) Homeotic gene Antennapedia mRNA contains 5'-noncoding sequences that confer translational initiation by internal ribosome binding. Genes. Dev. 6(9):1643-1653.

SEQ ID NO:2 is the IRES, name : Antp-DE (1323-1730, 408bp) as referenced in Oh S. K., Scott M. P., Sarnow P. (1992) Homeotic gene Antennapedia mRNA contains 5'-noncoding sequences that confer translational initiation by internal ribosome binding. Genes. Dev. 6(9):1643-1653, and Ye X., Fong P., Iizuka N., Choate D., Cavener D. R. (1997) Ultrabithorax and Antennapedia 5' untranslated regions promote developmentally regulated internal translation initiation. Mol. Cell. Biol. 17(3):1714-1721,

SEQ ID NO:3 is the IRES, name : Antp-CDE (1-1730, 1730bp) as referenced in Oh S. K., Scott M. P., Sarnow P. (1992) Homeotic gene Antennapedia mRNA contains 5'-noncoding sequences that confer translational initiation by internal ribosome binding. Genes. Dev. 6(9):1643-1653, and Ye X., Fong P., Iizuka N., Choate D., Cavener D. R. (1997) Ultrabithorax and Antennapedia 5' untranslated regions promote developmentally regulated internal translation initiation. Mol. Cell. Biol. 17(3):1714-1721,

SEQ ID NO:4 is the IRES from V00568 Human mRNA encoding the c-myc oncogene. IRES name : c-myc (1-395, 395bp) as referenced in Stoneley M., Paulin F. E., Le Quesne J. P., Chappell S. A., Willis A. E. (1998) C-Myc 5' untranslated region contains an internal ribosome entry segment. Oncogene. 16(3):423-428,

SEQ ID NO: 5 is the IRES from NM_005378 Homo sapiens v-myc myelocytomatosis viral related oncogene, neuroblastoma derived (avian)(MYCN), mRNA.

IRES name : N-myc (989-1308, 319bp) as referenced in
Jopling C. L., Willis A. E. (2001) N-myc translation is initiated via an internal ribosome entry segment that displays enhanced activity in neuronal cells. Oncogene. 20(21):2664-2670,

SEQ ID NO: 6 is the IRES from AF006822 Homo sapiens myelin transcription factor 2 (MYT2) mRNA, complete cds
IRES name : MYT2_997-1152 (997-1152, 156bp) as referenced in

Kim J. G., Armstrong R. C., Berndt J. A., Kim N. W., Hudson L. D. (1998) A secreted DNA-binding protein that is translated through an internal ribosome entry site (IRES) and distributed in a discrete pattern in the central nervous system. Mol. Cell. Neurosci. 12(3):119-140,

SEQ ID NO:7 is the IRES from AF013263 Homo sapiens apoptotic protease activating factor 1(Apaf-1) mRNA,complete cds. IRES name : Apaf-1 (345-577, 233bp) as referenced in Cold-well M. J., Mitchell S. A., Stoneley M., MacFarlane M., Willis A. E. (2000) Initiation of Apaf-1 translation by internal ribosome entry. Oncogene. 19(7):899-905,

SEQ ID NO: 8 is the IRES from AY752946 Human coxsackievirus B3 strain 20, complete genome. IRES name : CVB3 (1-750, 750bp) as referenced in Jang G. M., Leong L. E., Hoang L. T., Wang P. H., Gutman G. A., Semler B. L. (2004) Structurally distinct elements mediate internal ribosome entry within the 5'-noncoding region of a voltage-gated potassium channel mRNA. J. Biol. Chem. 279(46):47419-47430, and
Jimenez J., Jang G. M., Semler B. L., Waterman M. L. (2005) An internal ribosome entry site mediates translation of lymphoid enhancer factor-1. RNA. 11(9):1385-1399,

SEQ ID NO: 9 is the IRES from NC_001479 Encephalomyocarditis virus, complete genome. IRES name : EMCV (257-832, 576bp) as referenced in Wang Z., Weaver M., Magnuson N. S. (2005) Cryptic promoter activity in the DNA sequence corresponding to the pim-1 5'-UTR. Nucleic Acids Res. 33(7):2248-2258,

SEQ ID NO: 10 is the IRES from NC_003924 Cricket paralysis virus, complete genome IRES name : CrPV_5NCR (1-708, 708bp) as referenced in Wilson J. E., Powell M. J., Hoover S. E., Sarnow P. (2000) Naturally occurring dicistronic cricket paralysis virus RNA is regulated by two internal ribosome entry sites. Mol. Cell. Biol. 20(14):4990-4999,

SEQ ID NO: 11 is the IRES from NC_001461 Bovine viral diarrhea virus 1, complete genome IRES name : BVDVI_1-385 (1-385, 385bp) as cited in Chon S. K., Perez D. R., Donis R. O. (1998) Genetic analysis of the internal ribosome entry segment of bovine viral diarrhea virus. Virology. 251(2):370-382,

SEQ ID NO:12 is the IRES from Z46258 Hog cholera virus (Classical swine fever virus) 'Chinese' strain (C-strain; EP 0 351 901 B1) encoding polyprotein. IRES name : CSFV (1-373, 373bp) as referenced in Rijnbrand R., Bredenbeek P. J., Haasnoot P. C., Kieft J. S., Spaan W. J., Lemon S. M. (2001) The influence of downstream protein-coding sequence on internal ribosome entry on hepatitis C virus and other flavivirus RNAs. RNA. 7(4):585-597,

SEQ ID NO:13 is the IRES from NC 001655 Hepatitis GB virus B, complete genome RES name : GBV-B (1023-1467, 445bp) as referenced in Rijnbrand R., Abell G., Lemon S. M. (2000) Mutational analysis of the GB virus B internal ribosome entry site. J. Virol. 74(2):773-783.

SEQ ID NO:14 is the IRES from EMCV as used in the following examples, and

SEQ ID NO:15 is the IRES from EMCV as published in ww.iresite.org.

Moreover, reference is made to the following examples which are given to illustrate, not to limit the present invention.

### Examples

### Example 1

### Cell based assay of protein function and quantification of protein expression Agonist induced receptor internalisation

A cell based assay of protein function- such as agonist induced G-protein coupled receptor internalization - is devised and applied such that the desired activity of the protein can be measured or visualized and measured in a high content (visual) assay. Typically, for agonist induced receptor internalization, this may comprise the visual identification of cells where the receptor is internalized as part of a population of cells. This can be quantified by computer aided image recognition, for example. The assay can be using fusion proteins to the receptor that can be expressed in cells and directly visualized in a microscope, such as the green fluorescent protein (GFP). It can also be an assay where the endogenous cellular receptor is visualized by indirect methods such as immuno-labelling or using a fluorescent agonist. The assay cell line is typically stably transfected. In one embodiment, the receptor assay is visualized using 488 nM excitation and suitable emission filters.

The concept presents the following approach to examining the impact of protein expression (Target) on the assay. The target is expressed with a transcriptionally related reporter that is spectrally distinct from that used in the assay. This is designed such that there is a close to linear correlation between target expression and reporter expression. Methods to achieve this include the use of internal ribosomal entry sites, two identical activity promoters driving the target and reporter or two promoters where the activity is predictable. In the simplest case, the expression of the reporter is a measure/expression ruler to measure the expression of the target. Here, the present inventors describe the use of internal ribosomal entry sites.

The experiment is performed as follows: a target::reporter construct is transiently transfected into the assay cells in accordance with standard protocols (such as cationic lipid:DNA complex transfection, calcium phosphate mediated transfection, polymer based transfection methods, dendrimers as described in Sambrook and Russell, Molecular Cloning 3rd edition chapter 16, CSH press, 2001). These methods are not highly efficient and so a range of reporter expression is detected. This provides a range of cells expressing the target at measurable levels and thus the impact of expression can be determined by then visualizing the assay in cells expressing the target at different levels. Thus, a common drawback of cell transfection is used to advantage because of the cell based resolution of microscopy that permits identification of single cells. Thus, the assay is measured in cells that were not transfected and in those transfected and expressing the target at a range of concentrations in a single experimental image.

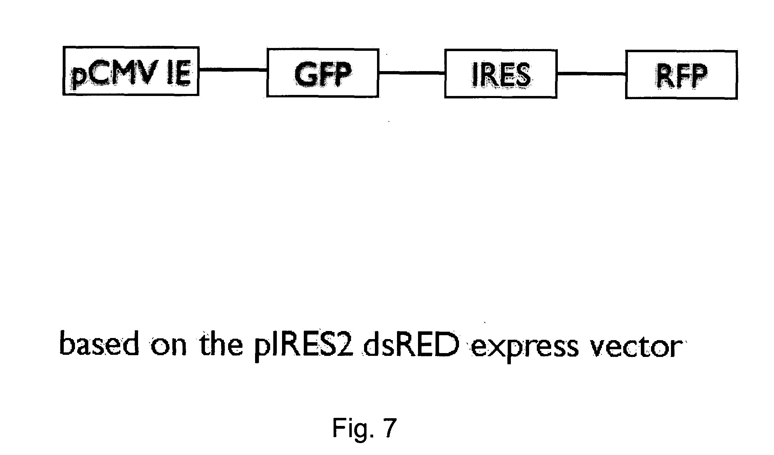

A copy of the green fluorescent protein was inserted into a internal ribosomal entry vector such that the IRES and the red fluorescent protein (dsRED express - a monomeric red fluorescent protein henceforth referred to as RFP) was downstream of it. (Figure 7) HEK293 cells were transiently transfected with this vector where expression of the inserts is controlled by the pCMV promoter (see also Figure 8) and then the fluorescent intensities of the green and red fluorescent protein were measured by confocal laser scanning microscopy of >25 cells per field of view using excitation (488/532nm) and detection optics suited to separately quantify the expression of the two fluorophores (Figure 1). The determined correlation coefficient was 0.91 +- 0.023 (n=5) between GFP and RFP intensity in confocal images of transfected cells, indicating that RFP or GFP expression can be used as a measure of expression of the other respective insert in the IRES vector.

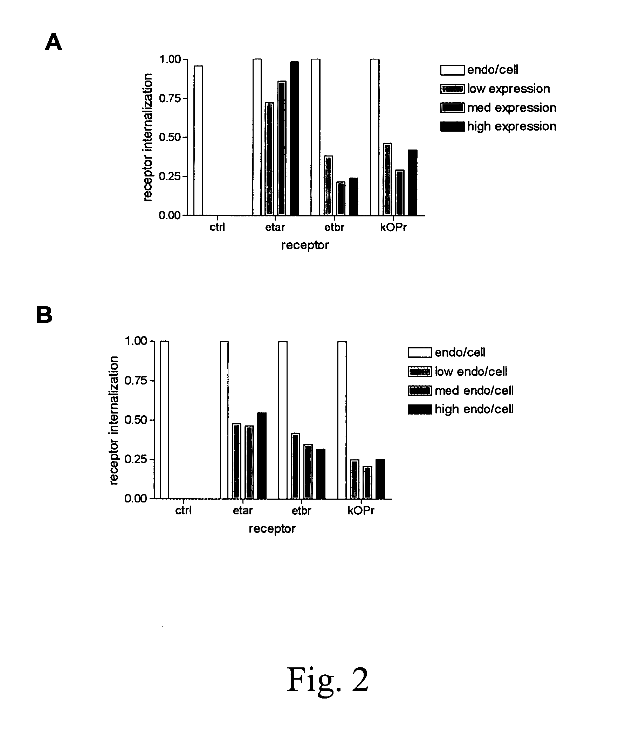

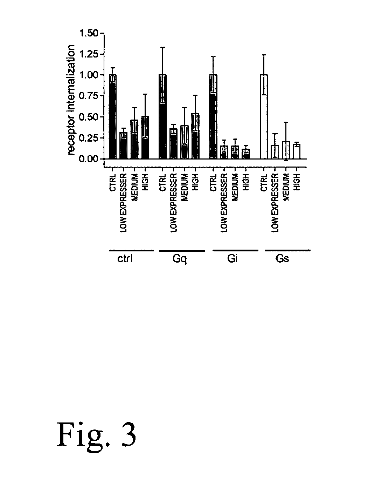

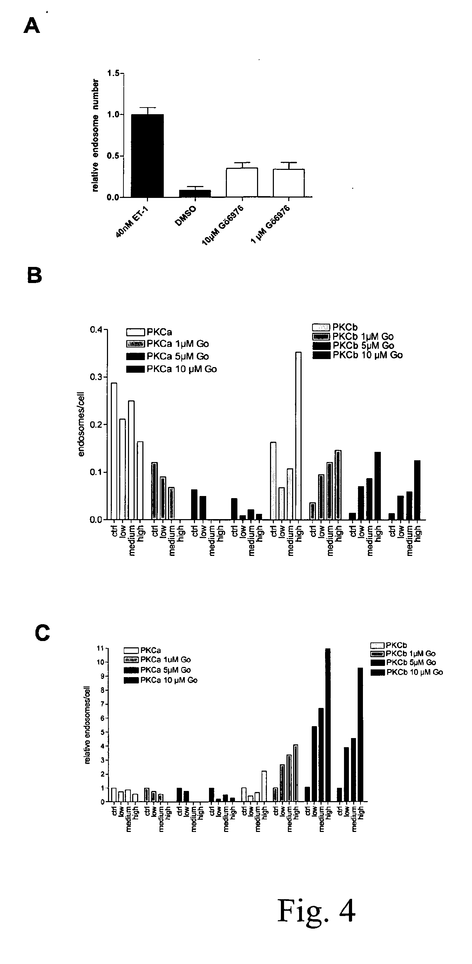

A series of cDNAs were cloned into the IRES:RFP vector encoding a) G-protein coupled receptors, b) small G-proteins or c) protein kinases. the sequence of the IRES used in these and all subsequent experiments is SEQ ID NO:14 which is an IRES from EMCV. Initially, the IRES vectors were transiently expressed in cell lines stably expressing a fusion protein between the endothelin A receptor and a c-terminal copy of the green fluorescent protein. Transfection efficiency into this line was on the order of 50% of cells by monitoring RFP expressing cells after transfection with IRES vectors. These vectors comprised either cDNAs encoding the endothelin B receptor, the kappa opioid receptor or the endothelin A receptor 5' to the IRES and the RFP. The effect of expression of the heterologous /external receptors on the agonist induced internalization of the endothelin A receptor was then measured. Two hours after cell stimulation with 40 nM endothelin-1, cells were imaged using an automated confocal microscope. Images of GFP labeled endothelin A receptor stably expressed in the cell lines could then be used to determine the fraction of cells where receptor endocytosis had occurred and the RFP image was used to determine the degree of endocytosis of the GFP-labelled endothelin A receptor in IRES vector transfected cells in the same image.
This was performed using both single blind manual counting and automated image analysis using custom journals in a commercial image analysis package (Metamorph offline, Molecular Devices, CA). Cells were identified as control (non-RFP-expresser) or expresser (RFP producer), and the expresser cells were further divided into low, medium and high expresser categories based on the integrated RFP intensity/ pixel in the images. The number of cells showing receptor internalization was automatically counted in these four categories and expressed as a fraction of the total number of cells in each category.
a) Three IRES vectors were separately transfected into the Endothelin A receptor-GFP cell line: the endothelin A receptor:IRES:RFP, the endothelin B receptor:IRES:RFP, and the kappa Opioid receptor :IRES:RFP. When compared to control cells in the same image, expression of the ETAR:IRES:RFP had minimal effects on ETAR-GFP internalization whereas both the Endothelin B receptor and the kappa Opioid receptor significantly reduced ETAR-GFP internalization (Figure 2A). This indicated that protein expression per se was a viable method for screening for proteins involved in a pathway.
b) To demonstrate that this method had selectivity across assays it was repeated using a kappa-Opioid receptor-GFP cell line, where no significant difference was determined in the assay for agonist induced opioid receptor internalization for any receptor expressed using the IRES vectors (Figure 2B).
   To further determine the usefulness of expression, IRES:RFP vectors were constructed for expression of three G-alpha protein components of heterotrimeric G-proteins, Gαs, Gαi, and Gαq. On expression in the ETAR-GFP cell line, two G-protein significantly reduced internalization (Gαs, Gαi) whereas Gq had no significant effect compared to control cells expressing RFP alone (Figure 3A).
   This demonstrates that the expression analysis strategy functions for receptors and small signalling proteins.
c) A series of vectors comprising either the protein kinase C isoforms, protein kinase C alpha, or protein kinase C beta inserted 5' to the IRES:RFP were transfected into the endothelin-A-receptor-GFP fusion protein stable cells lines. As shown in figure 4, expression of these protein kinase C isoforms caused no significant change in agonist induced internalization of the endothelin-A-receptor-fusion protein when compared to cells transfected with IRES:RFP vectors that possessed no 5' insert before the IRES and express only RFP.

### Example 2

### Identification of target proteins for small molecule modulators

The present inventors determined that the IRES approach could also be used for the identification of target proteins for compounds.

The agonist induced internalization of the endothelin A receptor is >85% blocked when cells are exposed to micro-molar concentrations of the compound Gö6976 (Figure 4A) which is a protein kinase C inhibitor (Martiny-Baron et al., 1993).
Protein kinase C has many isoforms and to determine whether the protein kinase C alpha or protein kinase C beta isoforms could be the targets of Gö6976 in the endothelin A receptor internalization assay, ETAR-GFP cells were transfected with IRES:RFP constructs bearing either protein kinase C alpha or protein kinase C beta.
Cells were transfected using standard methods (as reviewed in Sambrook and Russell, 2006), In particular lipofection using commercially available methods and protocols, such as Roche^{®} Fugene6, target systems targefect and its variance, lipofectamine etc. The cells- comprising both transfected and untransfected cells- were then exposed to a increasing concentration range of Gö6976, the cells were stimulated with the agonist endothelin-1. The number of endosomes per cell - as measure of Endothelin A receptor activity- was determined by semi-automated image analysis in the population of RFP expressing transfected cells compared to their control untransfected neighbouring cells.
The effect of expression of protein kinase C alpha in PKCα:IRES:RFP transfected cells was compared to the control untransfected cells in the same fields of view across the experimental concentration range of Gö6976 using automated confocal microscopy. Typically, cells were first exposed to a concentration range of Gö6976 for 120 min in 1% serum medium, then the medium was exchanged for medium containing identical concentrations of Gö6976 but supplemented with agonist (ten fold agonist EC50: 40 nM). After incubation, the cells were imaged using an automated high throughput confocal microscope (such as, the Opera from Evotec Technologies, Hamburg). Images defining the endothelin-A-receptor-GFP distribution were acquired as were images of the spectrally separate RFP expresser cells. The two color images per field of cells were aligned to correct for optical vignetting, mechanical misalignment (on the order of an XY displacement of 1-3µm; Metamorph Offline, Molecular devices corporation). non-RFP expresser cells were defined as the control population in the images and RFP expresser cells were segemtnedsegmented and then seperated into low, medium, and high (lower 20%, median, upper 20%) expressers based on the average RFP intensity per cell.
As expected, there was a significant decrease in the number of endosomes per cell in control cells as the concentration of Gö6976 was increased (Figure 4B left). A similar decrease was observed in cells expressing PKCα:IRES:RFP (Figure 4B left), indicating that protein kinase C alpha did not reverse the effect of the compound Gö6976. In contrast, the effect of expression of protein kinase C beta in PKCβ:IRES:RFP transfected cells was different. While increasing Gö6976 concentrations reduced the number of endosomes in control cells, endosomes were produced in PKCβ:IRES:RFP expressing cells even at the highest contractions of Gö6976 (Figure 4B). Thus, protein kinase C beta is identified as the target of Gö6976 in this assay for it provides 'gain of function' and reverses the phenotype and effec of Gö6976.
The identification of protein kinase C beta as the target of Gö6976 is robustly demonstrated when the numbers of endosomes are normalized to the control at each concentration (Figure 4C). As is clear in figure 4C, protein kinase C alpha expression and analysis using this system does not reverse the effect of Gö6976 while protein kinase C beta expression reverses the phenotype and is therefore the target of Gö6976 in the assay.

### Example 3

### Identification of target proteins for small molecules modulators using siRNA Nucleotransport of transcription factor nuclear factor kappa B

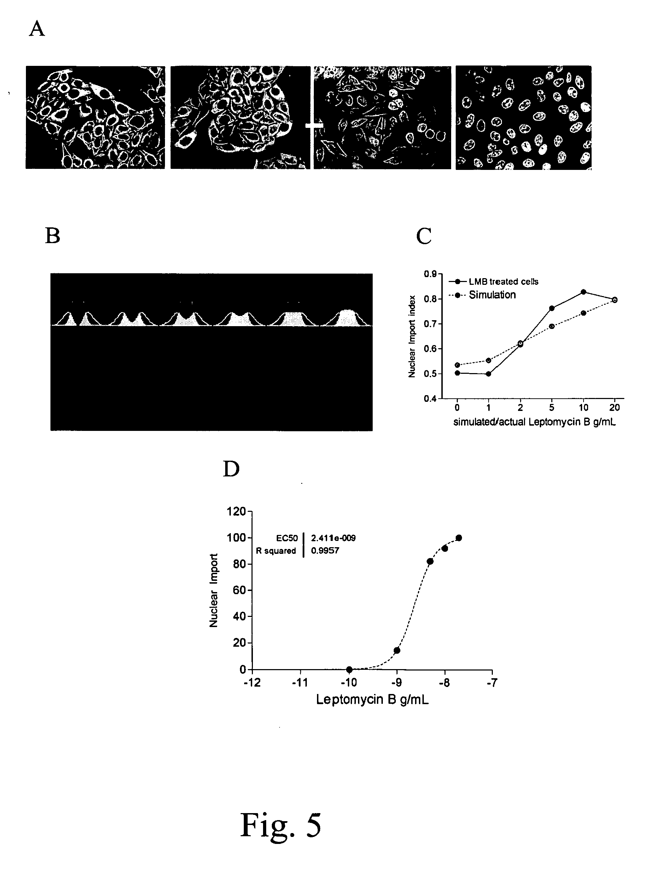

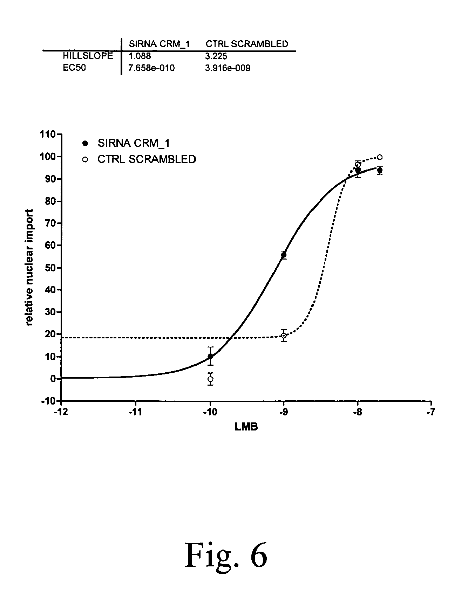

In addition to the method above which comprises gene expression as a 'gain for function' screen for drug targets, the present inventors also demonstrate a method where RNA interference is used to decrease protein expression and then determine if this is a target of a drug in question by a change in the AC50/IC50 concentration of the compound required to give 50% inhibition of the assay. This is a 'loss of function' screen.
In the case of nuclear transport as a proof of principle, the translocation of the transcription factor Nuclear factor kappa B was measured using high throughput immuno-fluorescent detection of the endogenously expressed NF-κB complex. Briefly, Hela cells were washed twice with PBS, then fixed for 10 minutes with 4% (w/v) paraformaldehyde in PBS, then washed with PBS. Permeabilization was performed with 0.1%TX-100 PBS for 10 minutes, cells were washed in PBS then incubated with 1:200 dilution of rabbit anti-NF-κB in 10% Goat serum-PBS overnight at 4°C. Plates were washed 3x with PBS for 10 min on an orbital rotator. 1:1000 Alexa-488 Goat anti-rabbit secondary antibody was incubated with the cells for 60 min at room temperature, cells were washed three times 10 minutes with PBS on orbital shaker before addition of 5µM DRAQ5 (DRAQ5 = nuclear stain) in PBS for 10 minutes at 37°C.
Leptomycin B was used to block nuclear transport and an IC₅₀ of 2 ng/mL or 4.4 nM was derived (Figure 5). The expression of the nuclear export protein exportin 1/CRM1 was reduced by the transfection of small inhibitory RNAs targeting the exportin 1 sequence and the effect of leptomycin B on NFkB transport was assessed as above. Exportin 1/CRM 1 knock down using siRNA was estimated to be 50% using the silencing of GFP in a GFP expresser stable cell line in parallel as a bench mark (not shown). The IC50 for leptomycin B in Exportin 1/CRM knock down cells was significantly reduced- by a factor of > 10 fold- as compared to control cells transfected with a scrambled siRNA with no known homology to human genes (Figure 6). Therefore, CRM 1 /exportin 1 is identified as the target for leptomycin B, as predicted from the literature (Fornerod et al., 1997), establishing that this method of target identification is viable, and would function in the larger context of genome wide screening.

More specifically, figure 5 shows Quantitation of NF-κB nucleo-cytoplasmic transport by a high throughput immuno-fluorescence assay. (**a**) Cytoplasmic NF-κB accumulates in the nucleus of HeLa cells treated with 0 (left panel), lng/mL (center panel) or 20 ng/mL leptomycin B for 40 min prior to fixation and detection with anti-NF-κB and Alexa 488 secondary antibody, and nuclear staining with 10µM Draq5. Scale bar 20 µm. (**b**) Simulated nuclear localization images with red representing the nucleus, green NF-κB localization where a cytoplasmic distribution of NF-κB is morphed onto the nucleus in an XZ (upper row) and XY (center row) simulated image series from left to right. The bottom row shows the labeling of cells after 0, 1, 5, 10, 20 ng/mL leptomycin B treatment with the green NF-κB labeling overlaid on the nuclear stain from left to right. Scale bar 5µM. (**c**) Quantitation of nuclear import on the simulation images (upper panel) and cell images (lower panel). (**d**) Determination of the EC50 for leptomycin in terms of nuclear localization of NF-κB after incubation with 0- 20 ng/mL Leptomycin B, detection of NF-κB and nuclear staining in microtitre plates. The fitted EC50 for leptomycin B was 2.4 ng/mL within 95% confidence interval of 1.9 to 3 ng/mL with an R squared of 0.9957 and is representative of >5 assays. All images were acquired on an automated confocal.

Figure 6 shows the quantitation of NF-κB nucleo-cytoplasmic transport by a high throughput immuno-fluorescence assay. Cells were transfected with either crm1 specific or scrambled siRNAs and leptomycin sensitive NFkB transport was measured after 24 hours. LMB curves were fitted using graphpad prism and had an R² >0.95. All images were acquired on an automated confocal. (LMB = leptomycin B).

### Example 4

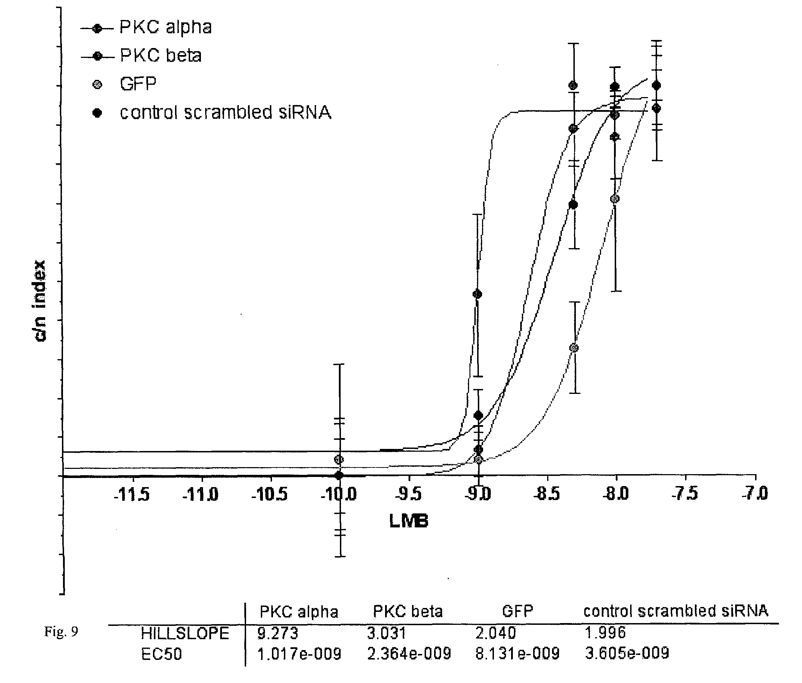

Figure 9 shows the effect of siRNA on the IC₅₀ of leptomycin B on the nuclear import of Nf kappa B when alternative siRNAs that are mechanistically irrelevant are silenced under the same conditions described above in example 3. As in example 3, the translocation of the transcription factor nuclear factor kappa B was measured using high throughput immunofluorescent detection of the endogenously expressed NF-kappa B Komplex. Briefly, Hela cells were washed twice with PBS, then fixed for 10 minutes with 4% (w/v) paraformaldehyde in PBS, then washed with PBS. Permeabilization was performed with 0.1%TX-100 PBS for 10 minutes, cells were washed in PBS then incubated with 1:200 dilution of rabbit anti-NF-κB in 10% Goat serum-PBS overnight at 4°C. Plates were washed 3x with PBS for 10 min on an orbital rotator. 1:1000 Alexa-488 Goat anti-rabbit secondary antibody was incubated with the cells for 60 min at room temperature, cells were washed three times 10 minutes with PBS on orbital shaker before addition of 5µM DRAQ5 (DRAQ5 = nuclear stain) in PBS for 10 minutes at 37°C.

Leptomycin B was used to block nuclear transport, and the IC₅₀ was measured. In the silencing experiments, scrambled siRNAs or siRNAs targeting the green fluorescent protein or protein kinase C isoforms were used. Silencing expression using control scrambled siRNAs, green fluorescent protein or protein kinase C isoforms is shown.

The data of figure 9 demonstrate the specificity that is achieved performing this experiment. Silencing a series of other cDNAs gives no phenotype and has no effect on the IC₅₀. The experiment of figure 9 was performed under the same silencing conditions as described in example 3.

### References

Echeverrri C.J. and Perrimon, N. (2006) High throughout RNAi in cultured cells:a user's guide. Nature reviews genetics 7, 374-384
Eggert, U.S. and Mitchison, T.J. (2006) Small molecule screening by imaging. Curr Opin Chem Biol. 2006, 10, 1-6
Fornerod, M., Ohno, M., Yoshida, M. and Mattaj, I.W. (1997) CRM1 is an export receptor for leucine-rich nuclear export signals. Cell, 90, 1051-1060.
Martiny-Baron, G., Kazanietz, M.G., Mischak, H., Blumberg, P.M., Kochs, G., Hug, H., Marme, D. and Schachtele, C. (1993) Selective inhibition of protein kinase C isozymes by the indolocarbazole Go 6976. J Biol Chem, 268, 9194-9197.
Peterson, J.R., Lebensohn, A.M., Pelish, H.E. and Kirschner, M.W. (2006) Biochemical suppression of small-molecule inhibitors: a strategy to identify inhibitor targets and signaling pathway components. Chem Biol, 13, 443-452.
Sambrook and Russell, Molecular Cloning 3rd, edition chapter 16, CSH press 2001

The features of the present invention disclosed in the specification, the claims and/or in the accompanying drawings, may, both separately, and in any combination thereof, be material for realizing the invention in various forms thereof.

## Claims

1. A method of detecting and/or quantifying expression of a target protein candidate in a cell comprising the steps:
- introducing a first nucleic acid encoding a marker protein into a vector,
- introducing a second nucleic acid encoding said target protein candidate the expression of which is to be detected and/or quantified, into said vector, such that said first and second nucleic acids are operably linked, such that expression of said marker protein is an indication of expression of said target protein candidate,
- introducing said vector into a cell,
- detecting and/or quantifying expression of said marker protein,
- relating said expression of said marker protein to expression of said target protein candidate, and thereby detecting and/or quantifying expression of said target protein candidate.

2. The method according to claim 1, wherein said first and second nucleic acids are operably linked within said vector by one of the following arrangements:
a) said first nucleic acid is under control of a first promoter, and said second nucleic acid is under control of a second promoter that is located separately from said first promoter, and said first and second promoters are identical in sequence or have identical activity,
b) said first nucleic acid is under control of a first promoter, and said second nucleic acid is under control of a second promoter that is located separately from said first promoter, and said first and second promoters are not identical in sequence, and the activities of each of said promoters are predictable,
c) said first and said second nucleic acids are under control of a single promoter, and said first and said second nucleic acids are separated from each other by a stretch of nucleotides containing an internal ribosome entry site (IRES).

3. The method according to any of the foregoing claims, wherein said marker protein is a fluorescent protein, a fragment of an antibody, an epitope, an enzyme, avidin, a peptide biotin mimic, a peptide that can be detected through direct binding, or by a peptide that can be detected through a chemical binding with or reaction with an organic molecule containing a chemical fluorophore or similar structure such that an optically detectable signal is produced.

4. The method according to any of the claims 2-3, wherein said IRES is selected from IRES from viruses, IRES from cellular mRNAs, in particular IRES from picornavirus, such as polio, EMCV and FMDV, flavivirus, such as hepatitis C virus (HCV), pestivirus, such as classical swine fever virus (CSFV), retrovirus, such as murine leukaemia virus (MLV), lentivirus, such as simian immunodifficiency virus (SIV), and insect RNA virus, such as cricket paralysis virus (CRPV), and IRES from cellular mRNAs, e.g. translation initiation factors, such as eIF4G, and DAP5, transcription factors, such as c-Myc, and NF-κB-repressing factor (NRF), growth factors, such as vascular endothelial growth factor (VEGF), fibroblast growth factor 2 (FGF-2), platelet-derived growth factor B (PDGF-B), homeotic genes, such as antennapedia, survival proteins, such as X-linked inhibitor of apoptosis (XIAP), and Apaf-1, and other cellular mRNA, such as BiP.

5. The method according to any of claims 2-4, wherein,
- if said first and second promoters are identical in sequence, they are selected from the group comprising CMV, EF1, SV40, human H1 and U6 promoters,
- if said first and second promoters have identical activity, each of them is independently selected from the group comprising CMV, EF1, SV40, human H1 and U6 promoters,
- if said first and second promoters are not identical in sequence and the activities of said promoters are predictable, each of said promoters is independently selected from the group comprising CMV, EF1, SV40, human H1 and U6 promoters
- said single promoter is selected from the group comprising CMV, EF1, SV40, human H1 and U6 promoters, and said IRES is selected from nucleic acids having a sequence selected from the group comprising SEQ ID NO:1-15.

6. The method according to any of the foregoing claims, wherein said introducing of said vector into said cell occurs by transformation, transfection, electroporation, viral transduction, transduction, ballistic delivery.

7. The method according to any of the foregoing claims, wherein said detecting and/or quantifying occurs by any optical detection method capable of quantitative measurement, in particular, an optical detection with a spatial resolution, microscopy, fluorescence activated cell sorting, UV-Vis spectrometry, fluorescence or phosphorescence measurements, bioluminescence measurements.

8. The method according to claim 7, wherein said microscopy is selected from the group comprising light microscopy bright field microscopy, polarization microscopy, fluorescence microscopy, in particular confocal fluorescence microscopy, evanescent wave excitation microscopy, fluorescence correlation spectroscopy, fluorescence life time microscopy, fluorescence cross correlation microscopy, fluorescence recovery after photobleaching microscopy, line scanning imaging, point scanning imaging, structured illumination, deconvolution microscopy, photon counting imaging.

9. The method according to any of the foregoing claims, wherein said target protein candidate and said marker protein are expressed in said cell as separate proteins.

10. A method of identifying a target protein of a small molecule modulator comprising the steps:
- providing a first cell of a type that is capable of producing a signal when said first cell is exposed to a small molecule modulator, wherein said signal is a signal that can be spatially resolved and, optionally, be quantified, preferably by microscopy,
- exposing said first cell to a small molecule modulator and spatially resolving and, optionally, quantifying a first signal that is produced by said first cell as response to said small molecule modulator,
- providing a second cell of the same type as said first cell and
- performing the method according to any of claims 1-9 on said second cell,
- during performance of the method according to any of claims 1-9 on said second cell, after introducing said vector into said second cell, exposing said second cell to said small molecule modulator, and spatially resolving and, optionally, quantifying a second signal that is produced by said second cell as response to said small molecule modulator,
- comparing said first signal with said second signal, and, if there is a difference between said first signal and said second signal, attributing said difference to the expression of said target protein candidate in said second cell, thereby identifying said target protein candidate as a target protein of said small molecule modulator.

11. The method according to claim 10, wherein said first signal and said second signal are optical signals that can be detected, spatially resolved, and, optionally, quantified, by microscopy.

12. The method according to claim 11, wherein said first signal and said second signal are fluorescence signals.

13. The method according to any of claims 10-12, wherein the expression of said marker protein produces a third signal that can be spatially resolved and distinguished from said first and second signals.

14. The method according to claim 13, wherein said third signal can be detected, spatially resolved and, optionally, quantified, by microscopy.

15. The method according to claim 14, wherein said third signal is a fluorescence signal, and said first and second signals are fluorescent signals, and said third signal is spectrally distinct from said first and second signals.

16. The method according to any of claims 11-15, wherein said first signal and said second signal can only be distinguished from each other by their respective quantity.

17. The method according to any of claims 10-16 which, for a given small molecule modulator, is performed with more than one, preferably a plurality of target protein candidates.

18. The method according to claim 17, which, for a given small molecule modulator, is performed with all possible target protein candidates of a genome of an organism.

19. The method according to any of claims 10-18 which is performed with more than one, preferably a plurality of small molecule modulators.

20. A method of identifying a target protein of a small molecule modulator comprising the steps:
- providing a first cell of a type that is capable of producing a signal when said cell is exposed to a small molecule modulator, wherein said signal is a signal that can be spatially resolved and, optionally, be quantified, preferably by microscopy,
- exposing said first cell to a small molecule modulator and spatially resolving and, optionally, quantifying a first signal that is produced by said first cell as response to said small molecule modulator, performing this step at a number of different concentrations of said small molecule modulator to determine a first half maximum active concentration (AC₅₀) of said small molecule modulator which is the half maximum active concentration in the absence of an inhibition of expression of said target protein candidate,
- determining said first half maximum active concentration,
- providing a second cell of the same type as said first cell and
- introducing into said second cell small inhibitory RNA (siRNA) that is selected so as to inhibit expression of a target protein candidate in said second cell, preferably transfecting said second cell using small inhibitory RNA (siRNA) that is selected so as to inhibit expression of a target protein candidate in said second cell,
- exposing said second cell to said small molecule modulator and spatially resolving and, optionally, quantifying a second signal that is produced by said second cell as response to said small molecule modulator, performing this step at a number of different concentrations of said small molecule modulator to determine a second half maximum active concentration (AC₅₀) of said small molecule modulator which is the half maximum active concentration in the presence of an inhibition of expression of said target protein candidate,
- determining said second half maximum active concentration,
- comparing said first half maximum active concentration with said second half maximum active concentration, and, if there is a difference between said first half maximum active concentration and said second half maximum active concentration, attributing said difference to the inhibition of expression of said target protein candidate, thereby identifying said target protein candidate as a target protein of said small molecule modulator.

21. The method according to claim 20, wherein said first and second half maximum active concentrations are half maximum inhibitory concentrations (IC₅₀), and said small molecule modulator is an inhibitor.

22. The method according to claim 20, wherein said first and second half maximum active concentrations are half maximum enhancing concentrations (EC₅₀) and said small molecule modulator is an enhancer.

23. The method according to any of claims 20-22, wherein said first signal and said second signal are optical signals that can be detected, spatially resolved, and, optionally quantified, by microscopy.

24. The method according to claim 23, wherein said first signal and said second signal are fluorescence signals.
